# EUROPEAN PATENT APPLICATION

(11) **EP 3 791 931 A1**
(43) Date of publication of application: **17.03.2021**
(21) Application number: 19197312.2
(22) Date of filing: 13.09.2019
(51) Int. Cl.: A61P 35/00, C07K 16/28, A61K 39/00

(54) **BISPECIFIC ANTIBODIES FOR THE TREATMENT OF SOLID TUMORS**

(71) Applicant: Ichnos Sciences SA, 2300 La Chaux-de-Fonds (CH)
(72) Inventor: REDDY, Venkateshwar, 2300 La Chaux-de-Fonds (CH)

(57) **Abstract**

The present invention relates to combinations of a T cell redirecting antibody and a second immuno-oncology or immunomodulatory agent to treat diseases, including cancer.

## Description

The present invention relates to methods of treating patients in need thereof by administering a bispecific antibody(ies) and in particular T cell redirecting bispecific antibodies to a patient suffering from a solid tumor.

### INTRODUCTION

CD38 is a 46-kDa membrane glycoprotein with a short 20-aa N-terminal sequence, a single transmembrane segment and a 256-aa catalytic carboxyl domain. It is a novel multifunctional molecule, serving not only as a differentiation antigen on cell surface, but also, amongst other functions, as the dominant signaling enzyme responsible for the metabolism of two intracellular calcium messenger molecules, cyclic ADP-ribose (cADPR) and nicotinic acid adenine dinucleotide phosphate (NAADP)).

In addition to its intracellular signaling functions, CD38 is also a surface antigen, serving as a receptor for ligands such as CD44 and CD316. CD38 is ubiquitously expressed in many cells, especially in the immune cells, such as lymphocytes and monocytes. CD38 expression is also extremely high in several malignant cells types, including multiple myeloma (MM), and chronic lymphoid leukemia. MM is a plasma-cell cancer characterized by accumulation of malignant cells in the bone marrow and production of a monoclonal immunoglobulin (M protein). MM remains incurable, although the median overall survival rate has been increased to 5 years owing to the introduction of novel treatments. Due to the differences in expression between normal and myeloma cells, CD38 is a validated drug target for MM therapy and the human monoclonal antibody, daratumumab (Darzalex™) has been approved by the FDA and EMA for the treatment of MM.

CD38-targeting antibodies are generally well tolerated and induce partial response or better in approximately 30% of heavily pretreated multiple myeloma (MM) patients as monotherapy.

Investigators have also had great success in treating other cancers with therapies which offset checkpoint inhibitors, such as CTLA-4 and PD-1. These remove *in vivo* inhibition of anti-tumor T cell responses through antibody-mediated antagonism of these receptors. It is increasingly clear however that removing the effects of one or more checkpoint inhibitor is not sufficient to promote tumor regression in a majority of patients. Generating a robust therapeutic immune response requires not only removing inhibitory pathways but also activating stimulatory pathways or combinations with other mechanisms to achieve a robust therapeutic immune response.

Within a tumour the presence of checkpoint inhibitors, can inhibit T cell function to suppress anti-tumor immune responses. Checkpoint inhibitors, such as CTLA-4 and PD-1, attenuate T cell proliferation and cytokine production. CD8 T cell responses also requires T cell receptor activation plus co-stimulation, which can be provided through ligation of tumor necrosis factor receptor family members, including OX40 (CD134) and 4-1BB (CD137). When used as single agents, these drugs can induce potent clinical and immunologic responses in patients with metastatic disease. However, each of these agents only benefits a subset of patients, highlighting the critical need for more effective combinatorial therapeutic strategies acting via more pathways/components of the immune system.

Unfortunately attempts in early-phase clinical trials to expand the number of patients who respond to daratumumab (Darzalex) in combination with either a PD-1 inhibitor for multiple myeloma or a PD-L1 inhibitor for non-small cell lung cancer (NSCLC) were terminated following a planned interim analysis. At a routine analysis of the CD38/PD-L1 combination study, an independent data monitoring committee (DMC) noted a lack of clinical efficacy with the combination compared with single-agent atezolizumab. Moreover, the DMC identified a higher rate of mortality in the daratumumab/atezolizumab combination arm.

Another class of immunotherapy agent are bispecific antibodies that activate and simultaneously bridge T cells (or other effector cells) to kill targeted tumor cells was conceived more than 30 years ago. Since then, two T-cell-redirecting bispecific antibodies (bsAbs) of different designs, catumaxomab and blinatumomab, have been approved by regulatory agencies, with many others at various stages of preclinical and clinical development. To date, the ongoing efforts to optimize the therapeutic potential of bispecific antibodies in general, and those intended to redirect immune cells, including T cells, NK cells, and Treg cells, in particular, have led to a plethora of functional constructs distinguishable by diverse formats and different specificities.

These bispecific therapies have shown efficacy in patients that do not respond to other therapies, but even in indications where efficacy has been established, various resistance mechanisms and non-responding patient sub-populations have been discovered and will likely be elucidated further as more patients receive these medicines.

Therefore although anti-CD38 antibodies and immuno-oncology medicines such as an anti-CTLA-4 antibody have allowed the treatment of previously untreatable patients, a large fraction of the patient population remain untreatable. This gap being due to the complexity of attempting to modulate the human immune system *per se* and in particular a patient's immune system which may be compromised due to the patent's general poor health and potential modulation by cancer cells or other factors associated with a disease, which allow the avoidance of detection and elimination by the patient's immune system.

Medicines that address these problems are therefore required.

### SUMMARY OF THE INVENTION

The present invention relates to a bispecific and in particular a T cell redirecting antibody to treat a solid tumor.

In accordance with the present invention a solid tumor is an abnormal mass of tissue that usually does not contain cysts or liquid areas. Solid tumors may be benign (not cancer), or malignant (cancer). Different types of solid tumors are named for the type of cells that form them. Examples of solid tumors are sarcomas, carcinomas, and lymphomas.

In particular the solid tumor may be selected from the group comprising head and neck squamous cell carcinoma, colorectal, prostate, and pancreatic or exocrine cancers.

Squamous cell carcinoma is a cancer that arises from particular cells called squamous cells. Squamous cells are found in the outer layer of skin and in the mucous membranes, which are the moist tissues that line body cavities such as the airways and intestines. Head and neck squamous cell carcinoma (HNSCC) develops in the mucous membranes of the mouth, nose, and throat.

HNSCC is classified by its location: it can occur in the mouth (oral cavity), the middle part of the throat near the mouth (oropharynx), the space behind the nose (nasal cavity and paranasal sinuses), the upper part of the throat near the nasal cavity (nasopharynx), the voicebox (larynx), or the lower part of the throat near the larynx (hypopharynx). Depending on the location, the cancer can cause abnormal patches or open sores (ulcers) in the mouth and throat, unusual bleeding or pain in the mouth, sinus congestion that does not clear, sore throat, earache, pain when swallowing or difficulty swallowing, a hoarse voice, difficulty breathing, or enlarged lymph nodes.

HNSCC can spread (metastasize) to other parts of the body, such as the lymph nodes or lungs. If it spreads, the cancer has a worse prognosis and can be fatal. About half of affected individuals survive more than five years after diagnosis.

Colorectal cancer (CRC), also known as bowel cancer and colon cancer, is the development of cancer from the colon or rectum (parts of the large intestine).

The present invention also provides a method for the treatment or prophylaxis of a disease or disorder which may be ameliorated by modulating a patient's immune system e.g., autoimmune, neurodegenerative, neurological, inflammatory, cancer, hyperproliferative, and cardiovascular diseases and disorders, comprising administering to a patient in need thereof an effective amount of bispecific and in particular a T cell redirecting antibody.

In accordance with another aspect of the present invention the T cell redirecting antibody is selected from the group comprising GBR 1302 (SEQ ID NOs: 1-6), GBR 1342 (SEQ ID NOs: 7-9), GBR 1372 (SEQ ID NOs: 10-13).

In accordance with the present invention the combination of a T cell redirecting antibody is suitable for treating a cancer characterised by the overexpression of a target antigen such as HER2, CD38 or EGRF and in particular selected from the group breast, ovarian, bladder, salivary gland, endometrial, pancreatic and non-small-cell lung cancer (NSCLC), breast carcinoma (invasive ductal & lobular, in situ, medullary), colorectal carcinoma (CRC) and polyps, prostate adenocarcinoma and hyperplasia of prostate, ovarian carcinoma and benign conditions, pancreatic adenocarcinoma, non-small cell lung carcinoma (including K-ras, EGFR status), melanoma (including B-raf status), renal cell carcinoma, brain tumors (glioblastoma, astrocytoma, medulloblastoma, etc.), head and neck squamous cell carcinoma, colorectal, prostate, and pancreatic or exocrine cancers

According to another aspect of the present invention, there is provided a method of treating a patient in need thereof using a bispecific antibody and in particular a T cell redirecting antibody, comprising administering the bispecific antibody, wherein said patient has at least one solid tumor.

### EXAMPLES

### Example 1 Materials and Methods

### 1.1 Procedure for detection of CD8 and Ki-67 by Immunohistochemistry

The basic general principle that underpins this technique and those detailed in 1.2 and 1.3, is the antigen-antibody reaction which is amplified and visualized. The target antigen may be physically inaccessible to the antibody due to protein folding caused during fixation. This is overcome by a procedure called antigen retrieval, where heat is used to alter the protein folding and the antigens become more accessible under a well-defined buffer condition. Quenching the endogenous peroxidase and protein block are important steps to avoid background staining and non-specific binding. This standardized protocol uses a three-layered detection system that involves the primary antibody (usually rabbit /mouse mAb or rabbit pAb) which binds to the target antigen; HRP-conjugated secondary antibody (usually goat anti-rabbit or anti mouse IgG, depending on compatibility) which binds the primary antibody. The antibodies aid detection of antigen and signal amplification. The peroxidase enzyme, which is present in the secondary antibody, catalyses a reaction where DAB (3,3'-diaminobenzidine) produces a relatively stable brown precipitate which can be visualized under a microscope, ultimately detecting the target antigen. The main advantage of the automation is the retrieval and staining protocols for standardization and staffing considerations and better reproducibility with the results obtained.

Three to four micron (3-4 µm) thick tissue sections are obtained from FFPE blocks on Tomo hydrophilic adhesion slides that offer superior tissue adhesion. Specimen Rejection Criteria: Qualifying status is determined depending on the adequacy of tumor in the histopathological examination. If there is no tumor or minimal tumor, the specimen is rejected for that particular study. Sample storage: FFPE blocks and slides can be stored for up to 10 years.

### Detailed protocol:

1. To start the run, empty the waste container according to the laboratory standards and replace it.
2. Prepare the bulk solutions according to the manufacturer's instructions.
3. Replace the bulk bottles in the desired slots. Note: DO NOT exchange the slots of the specified reagents.
4. Switch ON the power button and click Ventana icon.
5. Label the slides by clicking on the slide label icon at the bottom of the main screen. Select the protocol and enter the details such as Sample ID, Cancer Type, Study Name and date and print the labels.
6. To load the slides, open the slide tray and on the thermopad, load the slides with barcode away from the centre.
7. Before starting the run, load the reagents in the reagent rack and load it on the reagent compartment. Two secondary antibody kits should also be placed on the reagent rack (UltraView Universal DAB detection kit and UltraView Universal AP RED detection kit).
8. To start the run, click on the start button in the Ventana nexus software and enter the number of slides.
9. After dewaxing and cell conditioning instrument will give the alarm to add the primary antibody (Ki-67).
10. During next titration pop-up add the second primary antibody (CD8).
11. Add the antibody at 45° and close the compartment once done followed by pushing Start button on the bottom panel.
12. After the staining is done, dip the slides in two changes of 100% alcohol for 4 min.
13. Dip in xylene and then mount
14. Register reagents and dispensers appropriately every time a new lot is used.

**Table 1: Antibody specifications**

| S1 No. | Name of Antibody | Vendor | Catalogue No. | Dilution |
|---|---|---|---|---|
| 1 | CD8* | AbCam | ab4055 | 1:100 |
| 2 | Ki-67 | Dako | R626 | RTU |

| | | | | |
|---|---|---|---|---|
| *(HKi-67 will be stained brown and CD8 will be stained red. Proliferating CD8 cells will be quantified by a qualified pathologist). | | | | |

### 1.2 Procedure for Detection of CD38 by Immunohistochemistry

The aim of this method is to operate Benchmark GX (Roche) for performing automated IHC staining for CD38. The purpose of this SOP is to provide knowledge about the principle and steps involved in the immunohistochemical staining of tissue sections from FFPE blocks using Ventana Auto stainer for CD38. The study involves assessment of CD38 status at baseline in metastatic head and neck squamous cell carcinoma, colorectal, prostate, and pancreatic tumor specimens.

Three-Four micron (3-4µm) thick tissue sections are obtained from FFPE blocks on Tomo hydrophilic adhesion slides that offer superior tissue adhesion. Specimen Rejection Criteria: Qualifying status is determined depending on the adequacy of tumor in the histopathological examination. If there is no tumor or minimal tumor, the specimen is rejected for that particular study. Sample storage: FFPE blocks and slides can be stored for up to 10 years.

### Detailed protocol:

1. To start the run, empty the waste container according to the laboratory standards and replace it.
2. Prepare the bulk solutions according to the manufacturer's instructions.
3. Replace the bulk bottles in the desired slots. Note: DO NOT exchange the slots of the specified reagents.
4. Switch ON the power button and click Ventana icon.
5. Label the slides by clicking on the slide label icon at the bottom of the main screen. Select the protocol and enter the details such as Sample ID, Cancer Type, Study Name and Date, and print the labels.
6. To load the slides, open the slide tray and on the thermopad, load the slides with barcode away from the centre.
7. Before starting the run, load the reagents in the reagent rack and load the rack on the reagent compartment.
8. To start the run, click on the start button in the Ventana Nexus Software and enter the number of slides.
9. After dewaxing and cell conditioning, the instrument will sound the alarm to add the primary antibody (CD38).
10. Add the antibody at 45°, close the compartment, and push the Start button on the bottom panel.
11. After the staining is done, dip the slides in two changes of 100% alcohol for 4 min.
12. Dip in xylene and then mount.
13. Register reagents and dispensers appropriately every time for each new lot.

**Table 2: Antibody specifications**

| S1 No. | Name of Antibody | Vendor | Catalog No. | Dilution |
|---|---|---|---|---|
| 1 | CD38 (SP149) | Ventana Roche | 760-4785 | RTU |

### 1.3 Procedure for Detection of CD8 by Immunohistochemistry

This procedure is to operate Benchmark GX (Roche) for performing automated IHC staining for CD8. The purpose of this SOP is to provide knowledge about principle and steps involved in the immunohistochemical staining of tissue sections from FFPE blocks using Ventana Autostainer for CD8. The study involves assessment of CD8 status post-treatment with the suggested drugs and appropriate controls in metastatic head and neck squamous cell carcinoma, colorectal, prostate, and pancreatic tumor specimens.

Three-Four micron (3-4µm) thick tissue sections are obtained from FFPE blocks on Tomo hydrophilic adhesion slides that offer superior tissue adhesion. Specimen Rejection Criteria: Qualifying status is determined depending on the adequacy of tumor in the histopathological examination. If there is no tumor or minimal tumor, the specimen is rejected for that particular study. Sample storage: FFPE blocks and slides can be stored for up to 10 years.

### Detailed protocol:

1. To start the run, empty the waste container according to the laboratory standards and replace it.
2. Prepare the bulk solutions according to the manufacturer's instructions.
3. Replace the bulk bottles in the desired slots. Note: DO NOT exchange the slots of the specified reagents.
4. Switch ON the power button and click Ventana icon.
5. Label the slides by clicking on the slide label icon at the bottom of the main screen. Select the protocol and enter the details such as Sample ID, Cancer Type, Study Name and Date, and print the labels.
6. To load the slides, open the slide tray and on the thermopad, load the slides with barcode away from the centre.
7. Before starting the run, load the reagents in the reagent rack and load the rack on the reagent compartment.
8. To start the run, click on the start button in the Ventana Nexus Software and enter the number of slides.
9. After dewaxing and cell conditioning, the instrument will sound the alarm to add the primary antibody (CD8).
10. Add the antibody at 45°, close the compartment and push the Start button on the bottom panel.
11. After the staining is done, dip the slides in two changes of 100% alcohol for 4 min.
12. Dip in xylene and then mount.
13. Register reagents and dispensers appropriately every time a new lot is used.

**Table 3: Antibody specifications**

| S1 No. | Name of Antibody | Vendor | Catalogue No. | Dilution |
|---|---|---|---|---|
| 1 | CD8* | AbCam | ab4055 | 1:100 |

### 1.4 Canscript - M-Score

Mitra Biotech's (Woburn, Massachusetts, USA) CANscript platform uniquely provides a tumor model platform that preserves the native-state proliferation, morphology and viability of tumor cells within the context of the original TME. The platform consists of an ex vivo patient tumor culture model that uses intact tumor slices cultured with autologous plasma and autologous peripheral blood mononuclear cells.

By maintaining the complex structure, heterogeneity and behavior of tumors in culture, CANscript can be used to predict the response of individual patient tumors to monotherapies and combination therapies of many classes of drugs with high accuracy.

CANscript monitors a number of phenotypic readouts, both terminal and kinetic, including tumor cell proliferation, cell death, viability and tumor morphology. Data are analyzed using proprietary machine-learning algorithms that connect ex vivo data with clinical outcomes.

In the present invention 20 tumors of head and neck squamous cell carcinoma, 20 tumors of colorectal, 20 tumors of prostate, and 20 tumors of pancreatic cancer were obtained and cultured in the CANscript TME assay platform.

The study was performed as follows:

### Phase 1

Procurement of 20 tumors of head and neck squamous cell carcinoma, 20 tumors of colorectal, 20 tumors of prostate, and 20 tumors of pancreatic cancer.

Analyze vehicle control and drug-treated arms, and deliver M-Scores for all drug-treated arms for 80 samples. M-Score is a predictive tumor drug response indicator.

CD38+ status will be determined by IHC at baseline (T0) for all 80 samples.

CD8/Ki67 expression will be determined by IHC at T72 for all 80 samples.

### Phase 2

Once Phase 1 M-Score data from the first 40 samples has been reported, samples 1-40 will be further analyzed by Cytokine and NanoString analysis in Phase 2.

RNA profiling will be performed on selected samples, using the NanoString 10360 Gene Expression Panel at T72 on all study arms.

10-plex Cytokine/Chemokine analysis will be performed on selected samples from T24, T48 and T72 time points.

### Phase 3

Once Phase 1 M-Score data for Samples 41-80 has been reported, Glenmark will determine which samples will be further analyzed by Cytokine and NanoString analysis in Phase 3.

RNA profiling will be performed on selected samples using the NanoString 10360 Gene Expression Panel at T72 on all study arms.

10-plex Cytokine/Chemokine analysis will be performed on selected samples from T24, T48 and T72 time points.

RNA was isolated from FFPE tumor fragments in the vehicle arm (Rx1) in the absence of culture PBMC's. RNA applied to the NanoString PanCancer Immune Profiling panel. Normalized log2 Cell Scores shown as calculated using the nSolver Advanced Analysis software. All settings used default parameters with the exception of the "omit low count data" parameter which was manually set to 10.

The different treatment arms were as shown in Table 4.

**Table 4**

| **Samples** | **Treatment Arms (Rₓ)** | **Dose** |
|---|---|---|
| HNSCC, CRC, Prostate, and Pancreatic Cancer (n=20 each) | Rx1: Isotype Control | 15 ng/ml |
| | Rx2: GBR 1342 | 15 ng/ml |
| | Rx3: Daratumumab | 405.8 µg/ml |

### Example 1.5 - Tumor Score and Immune score

Tumor and Immune Score was calculated by the microscopy counting of the presence of NK Cells, Macrophages and B/T cells infiltrates in the tumor. The purpose of the immune score is to provide a qualitative assessment of the presence of immune cells in the isolated tumor, averaged across multiple fields and sections.

### Example 2. Results - M-Score

The inventors set out to undertake translational studies to identify responder and non-responders to GBR1342 in head and neck squamous cell carcinoma, colorectal, prostate, and pancreatic cancer, this study was also undertaken to evaluate the number of responder/non-responders in the same tumor to Daratumumab. Finally this study set out to identify differentiation features of CD3 engagers and in particular the induction of T cell memory responses, the induction of Effector T cells and elucidate mechanisms that tilt the balance between regulatory T cells vs Effector T cells.

This study tested GBR 1342 as a single agent (Rx2), , as well as in comparison to Daratumumab (Rx3), in human head and neck squamous cell carcinoma, colorectal, prostate, and pancreatic cancer cultured in the Mitra CANscript TME assay platform.

The first outcome of this study is a prediction, called the M-Score, of whether individual patient tumors would respond to the monotherapies (Rx2, Rx3).

The predicted response rate based on M-score (>25 is a predictive responder with 90% confidence to respond in clinic) of 1342 Monotherapy is based upon the following molecular basis, immune cell activation, potential immune mediated resistance mechanisms (allowing for further translational based development of other combinations), impact on Immune cell proliferation, impact of treatment on immuno-suppressive mechanisms.

The M-score is based on a proprietary trained alogorithm on ∼2000 patient samples predicting responder and non-responders and takes into account a composite scoring analysis which includes both kinetic and endpoint readouts. A M-Score >25 is considered a responder and has a 90% confidence to be a responder to the treatment in clinical setting.

A subset of the assays used to generate the score are shown in Table 5.

**Table 5**

| **PHENOTYPIC CELLULAR ASSAYS** | Biomarker and Detection Method |
|---|---|
| VALIDATED | |
| **Kinetic Assays** | |
| Proliferation | ATP Level |
| Proliferation | ATP/ADP Ratio |
| Proliferation | AmP Level |
| Proliferation | Cytochrome C |
| Metabolism | Glucose Utilization |
| Metabolism | Lactate |
| Metabolism | Pyruvate |
| Metabolism | Tryptophan |
| Metabolism | Protease |
| Death | Caspase 3 |
| Death | Caspase 7 |
| Death | Caspase 8 |

| **End Point Assays** | |
|---|---|
| Cell Viability | WST |
| Cell Viability | Prestoblue |
| Morphology | H-Score |
| Proliferation | Ki-67 |
| Death | Active Caspase 3 |
| Death | TUNEL |

The outcome for head and neck squamous cell carcinoma, is shown in Table 6 and Table 7. M-Score predicts the in-patient treatment outcome based on multiple input parameters for the given tumor specimen. Positive prediction of response: M-score >25 or M-score greater than 25. Negative prediction of response: M-score ≤ 25 or M-score of 25 or lower.

In summary out of 20 head and neck squamous cell carcinoma tumors, 10/20 tumors responded to at least one treatment arm (Rx2, 3). Maximum predicted efficacy (10/20 tumors (50%)) was observed in the GBR1342 arm (Rx2). 4 out of 20 tumors (20%) responded to Rx3 arm (Daratumumab).

The observed 20% response rate observed for Daratumumab, compares to published clinical data of Daratumumab of response rates between 10-20% in MM.

In Table 7, the Cumulative Analysis of CD8/Ki67 for head and neck squamous cell carcinoma tumors Tumors is presented. Ki-67 (also known as MKI67) is a cellular marker for proliferation and CD8 is a marker for cytotoxic T cells. Score for percentage positivity of CD8/Ki-67 dual IHC stained cells (averaged across multiple fields and sections) denotes proliferative cytotoxic T cells.

Predicted responders are shaded.

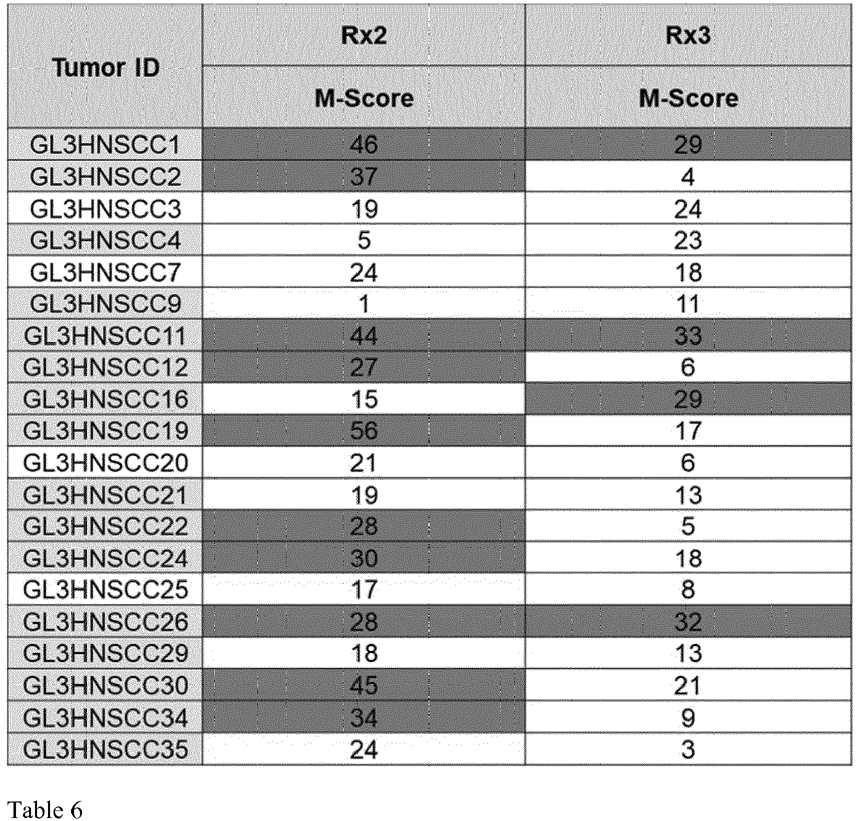

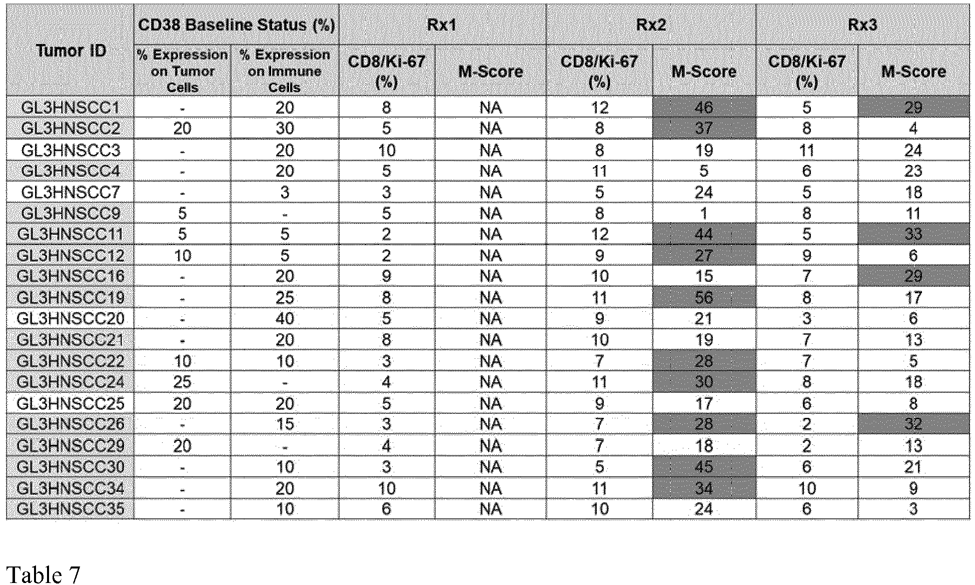

The outcome for colorectal cancer, is shown in Table 8 and Table 9. M-Score predicts the in-patient treatment outcome based on multiple input parameters for the given tumor specimen. Positive prediction of response: M-score >25 or M-score greater than 25. Negative prediction of response: M-score ≤ 25 or M-score of 25 or lower.

In summary out of 20 colorectal cancer tumors, 17/20 tumors responded to at least one treatment arm (Rx2, 3). Maximum predicted efficacy (16/20 tumors (80%)) was observed in the GBR1342 arm (Rx2). 8 out of 20 tumors (40%) responded to Rx3 arm (Daratumumab).

The observed 40% response rate observed for Daratumumab, is higher that the published clinical data of Daratumumab of response rates between 10-20% in MM.

In Table 9, the Cumulative Analysis of CD8/Ki67 for colorectal cancer is presented. Ki-67 (also known as MKI67) is a cellular marker for proliferation and CD8 is a marker for cytotoxic T cells. Score for percentage positivity of CD8/Ki-67 dual IHC stained cells (averaged across multiple fields and sections) denotes proliferative cytotoxic T cells.

Predicted responders are shaded.

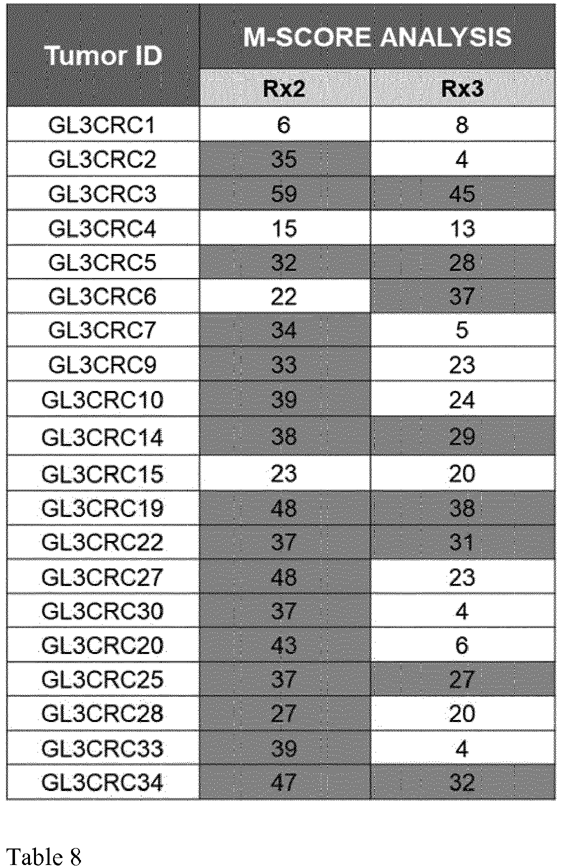

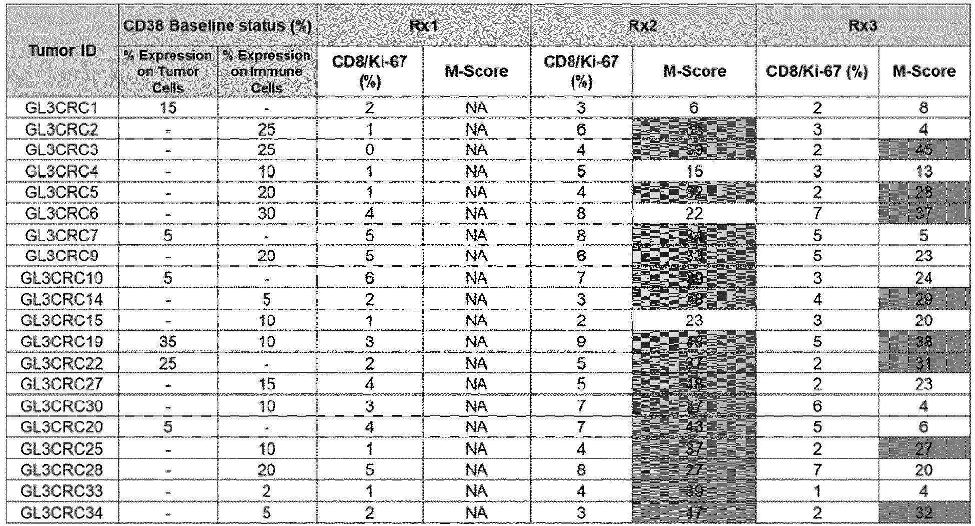

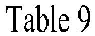

The outcome for prostate cancer, is shown in Table 10 and Table 11. M-Score predicts the in-patient treatment outcome based on multiple input parameters for the given tumor specimen. Positive prediction of response: M-score >25 or M-score greater than 25. Negative prediction of response: M-score ≤ 25 or M-score of 25 or lower.

In summary out of 20 prostate cancer tumors, 9/20 tumors responded to at least one treatment arm (Rx2, 3). Maximum predicted efficacy (9/20 tumors (45%)) was observed in the GBR1342 arm (Rx2). 3 out of 20 tumors (15%) responded to Rx3 arm (Daratumumab).

The observed 15% response rate observed for Daratumumab, compares to published clinical data of Daratumumab of response rates between 10-20% in MM.

In Table 11, the Cumulative Analysis of CD8/Ki67 for prostate cancer is presented. Ki-67 (also known as MKI67) is a cellular marker for proliferation and CD8 is a marker for cytotoxic T cells. Score for percentage positivity of CD8/Ki-67 dual IHC stained cells (averaged across multiple fields and sections) denotes proliferative cytotoxic T cells.

Predicted responders are shaded.

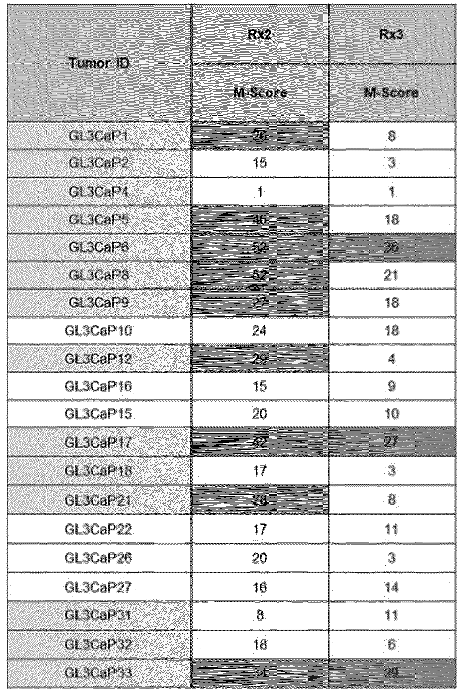

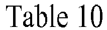

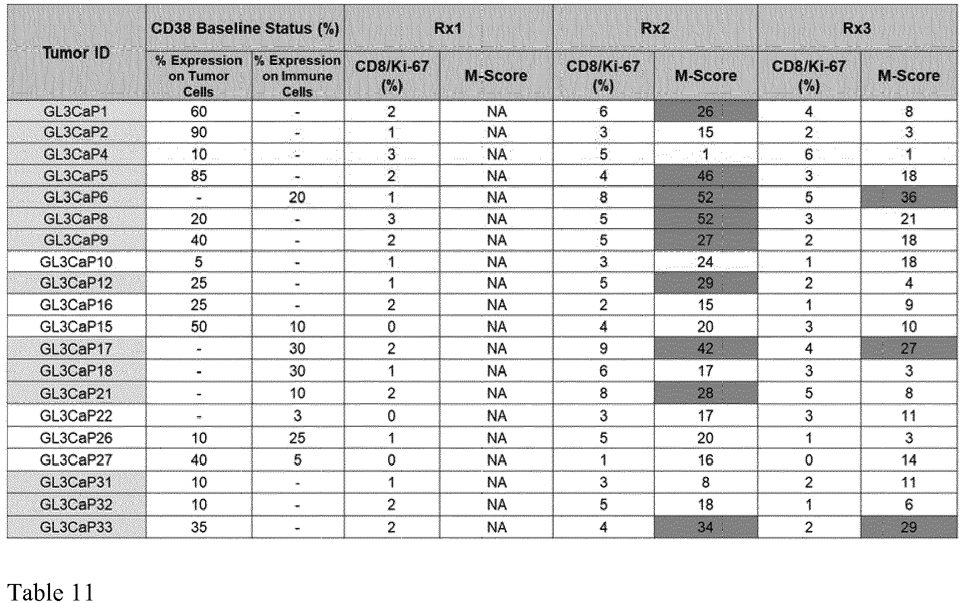

The outcome for pancreatic cancer, is shown in Table 12 and Table 13. M-Score predicts the in-patient treatment outcome based on multiple input parameters for the given tumor specimen. Positive prediction of response: M-score >25 or M-score greater than 25. Negative prediction of response: M-score ≤ 25 or M-score of 25 or lower.

In summary out of 15 pancreatic cancer tumors, 8/15 tumors responded to at least one treatment arm (Rx2, 3). Maximum predicted efficacy (6/15 tumors (40%)) was observed in the GBR1342 arm (Rx2). 5 out of 15 tumors (33%) responded to Rx3 arm (Daratumumab).

The observed 33% response rate observed for Daratumumab, compares to published clinical data of Daratumumab of response rates between 10-20% in MM.

In Table 12, the Cumulative Analysis of CD8/Ki67 for pancreatic cancer is presented. Ki-67 (also known as MKI67) is a cellular marker for proliferation and CD8 is a marker for cytotoxic T cells. Score for percentage positivity of CD8/Ki-67 dual IHC stained cells (averaged across multiple fields and sections) denotes proliferative cytotoxic T cells.

Predicted responders are shaded.

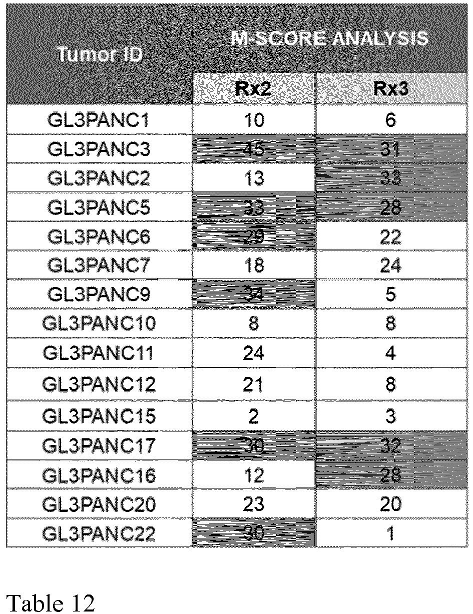

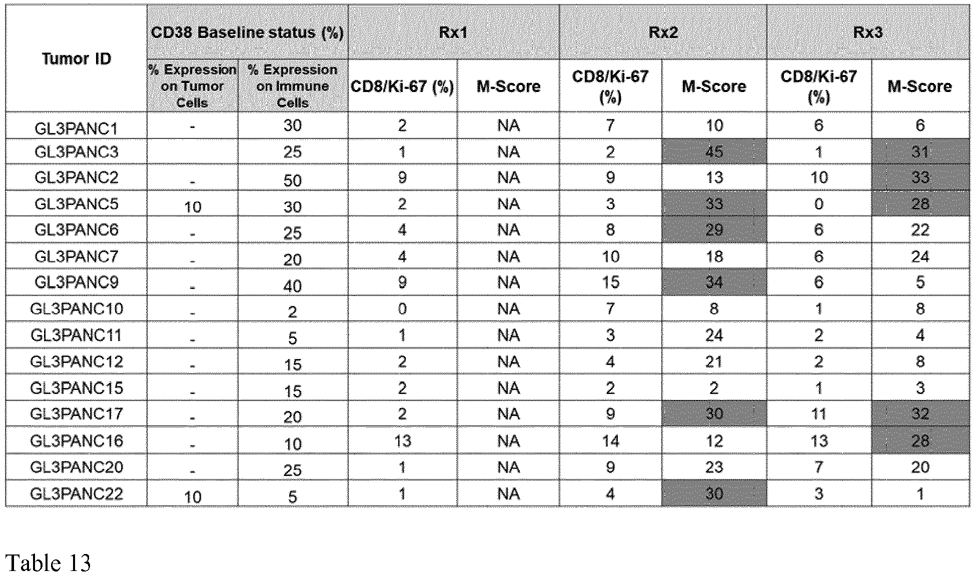

In Table 14, the Cumulative Analysis of CD8/Ki67 and CD38 status for HNSCC, CRC, Pancreas and Prostate Tumors are presented (N=45/75). Ki-67 (also known as MKI67) is a cellular marker for proliferation and CD8 is a marker for cytotoxic T cells. Score for percentage positivity of CD8/Ki-67 dual IHC stained cells (averaged across multiple fields and sections) denotes proliferative cytotoxic T cells.

Predicted responders are shaded.

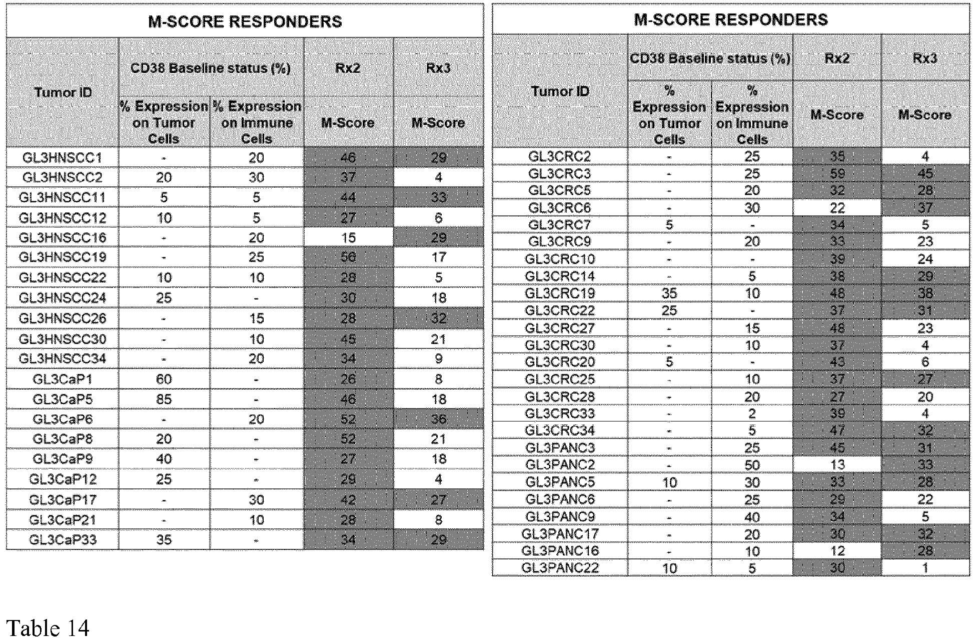

## Claims

1. AT cell redirecting antibody for use in treating a solid tumor.

2. AT cell redirecting antibody according to claim 1, selected from the group comprising GBR 1302 (SEQ ID NOs: 1-6), GBR 1342 (SEQ ID NOs: 7-9), GBR 1372 (SEQ ID NOs: 10-13).

3. AT cell redirecting antibody according to claim 1 or 2, wherein said solid tumor is selected from the group comprising head and neck squamous cell carcinoma, colorectal cancer, prostate cancer and pancreatic cancer.


SEQUENCE LISTING

<110>  Glenmark Pharmaceuticals SA
       Glenmark Pharmaceuticals S.A.

<120>  Bispecific Antibodies for the Treatment of Solid Tumors

<130>  GBR1342

<160>  12

<170>  PatentIn version 3.5

<210>  1
<211>  1491
<212>  DNA
<213>  Artificial Sequence

<220>
<223>  Herceptin ScFv LALA BTB Fc_DNA

<400>  1
atggagacag acacactcct gctatgggta ctgctgctct gggttccagg atccactggt      60

gaggtgcagc tggtcgagtc tggcggcgga ctggtgcagc ctggcggctc cctgcggctg     120

tcctgcgccg cctccggctt caacatcaag gacacctaca tccactgggt gcggcaggcc     180

cctggcaagg gcctggagtg ggtggcccgg atctacccta ccaacggcta caccagatac     240

gccgactccg tgaagggccg gttcaccatc tccgccgaca cctccaagaa caccgcctac     300

ctgcagatga actccctgcg ggccgaggac accgccgtgt actactgctc cagatgggga     360

ggagatggct tctacgccat ggactactgg ggccagggca ccctggtgac cgtgtcctcc     420

ggtggcggtg gcagcggcgg tggtggttcc ggaggcggcg gttctgacat ccagatgacc     480

cagtccccct ccagcctgtc tgcctccgtg ggcgaccggg tgaccatcac ctgccgggcc     540

tcccaggacg tgaacaccgc cgtggcctgg tatcagcaga agcctggcaa ggcccctaag     600

ctgctgatct actccgcctc cttcctgtac tccggcgtgc cttcccggtt ctccggctcc     660

cggtccggca ccgacttcac cctgaccatc tcctccctgc agcctgagga cttcgccacc     720

tactactgcc agcagcacta caccacccct cctaccttcg gccagggcac caaggtggag     780

atcaagaggg gaggaggagg taccgacaaa actcacacat gcccaccgtg cccagcacct     840

gaagccgccg ggggaccgtc agtcttcctc ttccccccaa aacccaagga caccctcatg     900

atctcccgga cccctgaggt cacatgcgtg gtggtggacg tgagccacga agaccctgag     960

gtcaagttca actggtacgt ggacggcgtg gaggtgcata atgccaagac aaagccgcgg    1020

gaggagcagt acaacagcac gtaccgtgtg gtcagcgtcc tcaccgtcct gcaccaggac    1080

tggctgaatg gcaaggagta caagtgcaag gtctccaaca aagccctccc agcccccatc    1140

gagaaaacca tctccaaagc caaaggacag cccagggaac ctgaggtggc cacattccca    1200

cctagccggg acgagctgac aaaaaatcag gtcaccctcg tctgtctcgt gaccggcttt    1260

tacccttccg acattgccgt ggaatgggaa tccaatgggc agcccgagaa caattacaag 1320

acagaccccc ccctgctgga atccgatggc agcttcgccc tgagcagccg gctgcgggtg 1380

gacaagtcca gatggcagca ggggaatgtc ttttcctgct ccgtcatgca tgaagccctc 1440

cacaatcatt atacacagaa aagcctgagc ctgtctcctg gcaagtgata a 1491

<210> 2
<211> 495
<212> PRT
<213> Artificial Sequence

<220>
<223> Herceptin ScFv LALA BTB Fc_Protein

<400> 2

Met Glu Thr Asp Thr Leu Leu Leu Trp Val Leu Leu Leu Trp Val Pro
1               5                   10                  15

Gly Ser Thr Gly Glu Val Gln Leu Val Glu Ser Gly Gly Gly Leu Val
            20                  25                  30

Gln Pro Gly Gly Ser Leu Arg Leu Ser Cys Ala Ala Ser Gly Phe Asn
        35                  40                  45

Ile Lys Asp Thr Tyr Ile His Trp Val Arg Gln Ala Pro Gly Lys Gly
    50                  55                  60

Leu Glu Trp Val Ala Arg Ile Tyr Pro Thr Asn Gly Tyr Thr Arg Tyr
65                  70                  75                  80

Ala Asp Ser Val Lys Gly Arg Phe Thr Ile Ser Ala Asp Thr Ser Lys
                85                  90                  95

Asn Thr Ala Tyr Leu Gln Met Asn Ser Leu Arg Ala Glu Asp Thr Ala
            100                 105                 110

Val Tyr Tyr Cys Ser Arg Trp Gly Gly Asp Gly Phe Tyr Ala Met Asp
        115                 120                 125

Tyr Trp Gly Gln Gly Thr Leu Val Thr Val Ser Ser Gly Gly Gly Gly
    130                 135                 140

Ser Gly Gly Gly Gly Ser Gly Gly Gly Gly Ser Asp Ile Gln Met Thr
145                 150                 155                 160

Gln Ser Pro Ser Ser Leu Ser Ala Ser Val Gly Asp Arg Val Thr Ile
                165                 170                 175

Thr Cys Arg Ala Ser Gln Asp Val Asn Thr Ala Val Ala Trp Tyr Gln
        180                 185                 190

Gln Lys Pro Gly Lys Ala Pro Lys Leu Leu Ile Tyr Ser Ala Ser Phe
    195                 200                 205

Leu Tyr Ser Gly Val Pro Ser Arg Phe Ser Gly Ser Arg Ser Gly Thr
210                 215                 220

Asp Phe Thr Leu Thr Ile Ser Ser Leu Gln Pro Glu Asp Phe Ala Thr
225                 230                 235                 240

Tyr Tyr Cys Gln Gln His Tyr Thr Thr Pro Pro Thr Phe Gly Gln Gly
                245                 250                 255

Thr Lys Val Glu Ile Lys Arg Gly Gly Gly Gly Thr Asp Lys Thr His
            260                 265                 270

Thr Cys Pro Pro Cys Pro Ala Pro Glu Ala Ala Gly Gly Pro Ser Val
        275                 280                 285

Phe Leu Phe Pro Pro Lys Pro Lys Asp Thr Leu Met Ile Ser Arg Thr
    290                 295                 300

Pro Glu Val Thr Cys Val Val Val Asp Val Ser His Glu Asp Pro Glu
305                 310                 315                 320

Val Lys Phe Asn Trp Tyr Val Asp Gly Val Glu Val His Asn Ala Lys
                325                 330                 335

Thr Lys Pro Arg Glu Glu Gln Tyr Asn Ser Thr Tyr Arg Val Val Ser
            340                 345                 350

Val Leu Thr Val Leu His Gln Asp Trp Leu Asn Gly Lys Glu Tyr Lys
        355                 360                 365

Cys Lys Val Ser Asn Lys Ala Leu Pro Ala Pro Ile Glu Lys Thr Ile
    370                 375                 380

Ser Lys Ala Lys Gly Gln Pro Arg Glu Pro Glu Val Ala Thr Phe Pro
385                 390                 395                 400

Pro Ser Arg Asp Glu Leu Thr Lys Asn Gln Val Thr Leu Val Cys Leu
                405                 410                 415

Val Thr Gly Phe Tyr Pro Ser Asp Ile Ala Val Glu Trp Glu Ser Asn
            420                 425                 430

Gly Gln Pro Glu Asn Asn Tyr Lys Thr Asp Pro Pro Leu Leu Glu Ser
        435                 440                 445

Asp Gly Ser Phe Ala Leu Ser Ser Arg Leu Arg Val Asp Lys Ser Arg
    450                 455                 460

Trp Gln Gln Gly Asn Val Phe Ser Cys Ser Val Met His Glu Ala Leu
465                 470                 475                 480

His Asn His Tyr Thr Gln Lys Ser Leu Ser Leu Ser Pro Gly Lys
                485                 490                 495

<210>  3
<211>  1413
<212>  DNA
<213>  Artificial Sequence

<220>
<223>  OKT3 H11 1133 LALA BTA_DNA

<400>  3
atggagacag acacactcct gctatgggta ctgctgctct gggttccagg atccactggt      60

gaagtgcagc tggtggaatc tggcggcgga ctggtgcagc ctggcggatc tctgagactg     120

agctgtgccg ccagcggcta caccttcacc cggtacacaa tgcactgggt gcgccaggcc     180

cctggcaagg gactggaatg gatcggctac atcaacccca gccggggcta cacctactac     240

gccgatagcg tgaagggccg gttcaccctc agcaccgaca agagcaagaa caccgcctac     300

ctgcagatga gctccctgcg ggccgaggac accgccgtgt actactgcgc ccggtactac     360

gacgaccact actgcctgga ctactggggc cagggcacac tcgtgacagt gtctagcgcg     420

tcgaccaagg gccccagcgt gttcccgcta gcccccagca gcaagagcac cagcggcggc     480

acagccgccc tgggctgcct ggtgaaggac tacttccccg agcccgttac cgtgtcctgg     540

aactctggag ccctgacctc cggcgtgcac accttccccg ccgtgctcca gagcagcggc     600

ctgtacagcc tgagcagcgt ggtgacagtg cccagcagca gcctgggaac ccagacctac     660

atctgcaacg tgaaccacaa gcccagcaac accaaggtgg acaagaaggt ggagcccaag     720

agctgcgaca aaactcacac ctgcccaccc tgccctgccc ctgaggccgc cggcggaccc     780

tccgtgttcc tgttcccccc aaagcccaag gacaccctga tgatcagccg gacccccgaa     840

gtgacctgcg tggtggtgga tgtgtcccac gaggaccccg aggtgcagtt caagtggtac     900

gtggacggcg tggaagtgca caacgccaag accaagccca gagaggaaca gtacaacagc     960

accttccggg tggtgtccgt gctgaccgtg ctgcaccagg actggctgaa cggcaaagag    1020

tacaagtgca aggtgtccaa caaggccctg cctgccccca tcgagaaaac catcagcaag    1080

acaaagggcc agccccgcga gcccgccgtg tacacactgc ctccaagccg ggaagagatg 1140

accaagaacc aagtgaagct cgtgtgcctc gtgaccggct tctacccctc cgatatcgcc 1200

gtggaatggg agagcagcgg ccagcccgag aacaactact acaccacccc ccccatgctg 1260

gacagcgacg gctcttttag cctggtgtcc tggctgaatg tggacaagag ccggtggcag 1320

cagggcaaca tcttcagctg cagcgtgatg cacgaggccc tgcacaaccg gttcacccag 1380

aagtccctga gcctgtctcc tggcaagtga taa 1413

<210> 4
<211> 469
<212> PRT
<213> Artificial Sequence

<220>
<223> OKT3 H11 1133 LALA BTA_Protein

<400> 4

Met Glu Thr Asp Thr Leu Leu Leu Trp Val Leu Leu Leu Trp Val Pro
1               5                   10                  15

Gly Ser Thr Gly Glu Val Gln Leu Val Glu Ser Gly Gly Gly Leu Val
            20                  25                  30

Gln Pro Gly Gly Ser Leu Arg Leu Ser Cys Ala Ala Ser Gly Tyr Thr
        35                  40                  45

Phe Thr Arg Tyr Thr Met His Trp Val Arg Gln Ala Pro Gly Lys Gly
    50                  55                  60

Leu Glu Trp Ile Gly Tyr Ile Asn Pro Ser Arg Gly Tyr Thr Tyr Tyr
65                  70                  75                  80

Ala Asp Ser Val Lys Gly Arg Phe Thr Leu Ser Thr Asp Lys Ser Lys
                85                  90                  95

Asn Thr Ala Tyr Leu Gln Met Ser Ser Leu Arg Ala Glu Asp Thr Ala
            100                 105                 110

Val Tyr Tyr Cys Ala Arg Tyr Tyr Asp Asp His Tyr Cys Leu Asp Tyr
        115                 120                 125

Trp Gly Gln Gly Thr Leu Val Thr Val Ser Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Ala
                245                 250                 255

Ala Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Gln Phe Lys Trp Tyr Val Asp Gly Val
    290                 295                 300

Glu Val His Asn Ala Lys Thr Lys Pro Arg Glu Glu Gln Tyr Asn Ser
305                 310                 315                 320

Thr Phe Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Thr Lys Gly Gln Pro Arg Glu Pro
        355                 360                 365

Ala Val Tyr Thr Leu Pro Pro Ser Arg Glu Glu Met Thr Lys Asn Gln
    370                 375                 380

Val Lys Leu Val Cys Leu Val Thr Gly Phe Tyr Pro Ser Asp Ile Ala
385                 390                 395                 400

Val Glu Trp Glu Ser Ser Gly Gln Pro Glu Asn Asn Tyr Tyr Thr Thr
                405                 410                 415

Pro Pro Met Leu Asp Ser Asp Gly Ser Phe Ser Leu Val Ser Trp Leu
            420                 425                 430

Asn Val Asp Lys Ser Arg Trp Gln Gln Gly Asn Ile Phe Ser Cys Ser
        435                 440                 445

Val Met His Glu Ala Leu His Asn Arg Phe Thr Gln Lys Ser Leu Ser
    450                 455                 460

Leu Ser Pro Gly Lys
465

<210>  5
<211>  699
<212>  DNA
<213>  Artificial Sequence

<220>
<223>  OKT3 L8 LC_DNA

<400>  5
atggagacag acacactcct gctatgggta ctgctgctct gggttccagg atccactggt      60

gatatccagc tgacccagag ccccagcagc ctgtctgcca gcgtgggcga cagagtgacc     120

atcacctgta gagccagcag cagcgtgtcc tacgtggcct ggtatcagca gaagcccggc     180

aaggccccca agaggtggat ctacgacacc tccaagctgt actccggcgt gcccagcaga     240

ttcagcggca gcggatccgg caccgactac accctgacca tcagctccct gcagcccgag     300

gacttcgcca cctactactg ccagcagtgg tccagcaacc cccccacatt tggacagggc     360

accaaggtgg aaatcaagcg tacggtggcc gctcccagcg tgttcatctt cccccccagc     420

gacgagcagc tgaagagcgg caccgcctcc gtggtgtgcc tgctgaacaa cttctacccc     480

cgggaggcca aggtgcagtg gaaggtggac aacgccctcc agagcggcaa cagccaggaa     540

agcgtcaccg agcaggacag caaggactcc acctacagcc tgagcagcac cctgaccctg     600

agcaaggccg actacgagaa gcacaaggtg tacgcctgcg aggtgaccca ccagggcctg     660

tccagccccg tgaccaagag cttcaaccgg ggcgagtgc                            699

<210>  6
<211>  233
<212>  PRT
<213>  Artificial Sequence

<220>
<223>  OKT3 L8 LC_Protein

<400>  6

Met Glu Thr Asp Thr Leu Leu Leu Trp Val Leu Leu Leu Trp Val Pro
1               5                   10                  15

Gly Ser Thr Gly Asp Ile Gln Leu Thr Gln Ser Pro Ser Ser Leu Ser
            20                  25                  30

Ala Ser Val Gly Asp Arg Val Thr Ile Thr Cys Arg Ala Ser Ser Ser
        35                  40                  45

Val Ser Tyr Val Ala Trp Tyr Gln Gln Lys Pro Gly Lys Ala Pro Lys
    50                  55                  60

Arg Trp Ile Tyr Asp Thr Ser Lys Leu Tyr Ser Gly Val Pro Ser Arg
65                  70                  75                  80

Phe Ser Gly Ser Gly Ser Gly Thr Asp Tyr Thr Leu Thr Ile Ser Ser
                85                  90                  95

Leu Gln Pro Glu Asp Phe Ala Thr Tyr Tyr Cys Gln Gln Trp Ser Ser
            100                 105                 110

Asn Pro Pro Thr Phe Gly Gln Gly Thr Lys Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230

<210>  7
<211>  234
<212>  PRT
<213>  Artificial Sequence

<220>
<223>  GBR1342 light chain sequence (hum9G7_VL_cK)

<400>  7

Met Arg Ser Pro Ala Gln Leu Leu Phe Leu Leu Leu Leu Trp Ile Pro
1               5                   10                  15

Gly Thr Asn Ala Asp Ile Gln Met Thr Gln Ser Pro Ser Ser Leu Ser
            20                  25                  30

Ala Ser Val Gly Asp Arg Val Thr Ile Thr Cys Gln Ala Ser Gln Asp
        35                  40                  45

Val Ile Thr Ser Val Ala Trp Tyr Gln Gln Lys Pro Gly Lys Ala Pro
    50                  55                  60

Lys Leu Leu Ile Tyr Ser Ala Ser Tyr Arg Tyr Thr Gly Val Pro Ser
65                  70                  75                  80

Arg Phe Ser Gly Ser Gly Ser Gly Thr Asp Phe Thr Phe Thr Ile Ser
                85                  90                  95

Ser Leu Gln Pro Glu Asp Ile Ala Thr Tyr Tyr Cys Gln Gln His Tyr
            100                 105                 110

Thr Ile Pro Leu Thr Phe Gly Gln Gly Thr Lys Leu Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  8
<211>  470
<212>  PRT
<213>  Artificial Sequence

<220>
<223>  GBR1342 heavy chain sequence (h9G7 1133 BTA LALA FTO (dA))

<400>  8

Met Arg Ser Pro Ala Gln Leu Leu Phe Leu Leu Leu Leu Trp Ile Pro
1               5                   10                  15

Gly Thr Asn Ala Gln Val Thr Leu Lys Glu Ser Gly Pro Thr Leu Val
            20                  25                  30

Lys Pro Thr Gln Thr Leu Thr Leu Thr Cys Thr Phe Ser Gly Leu Ser
        35                  40                  45

Leu Ser Thr Ser Gly Lys Gly Val Gly Trp Ile Arg Gln Pro Pro Gly
    50                  55                  60

Lys Ala Leu Glu Trp Leu Ala His Ile Trp Trp Asp Asp Asp Lys Arg
65                  70                  75                  80

Tyr Asn Pro Ala Leu Lys Ser Arg Leu Thr Ile Thr Lys Asp Thr Ser
                85                  90                  95

Lys Asn Gln Val Val Leu Thr Met Thr Asn Met Asp Pro Val Asp Thr
            100                 105                 110

Ala Thr Tyr Tyr Cys Ala Arg Ile Glu Leu Gly Arg Ser Tyr Val Met
        115                 120                 125

Asp Tyr Trp Gly Gln Gly Thr Leu Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Val Glu
225                 230                 235                 240

Pro Lys Ser Cys Asp Lys Thr His Thr Cys Pro Pro Cys Pro Ala Pro
                245                 250                 255

Glu Ala Ala Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Gln Phe Lys Trp Tyr Val Asp
    290                 295                 300

Gly Val Glu Val His Asn Ala Lys Thr Lys Pro Arg Glu Glu Gln Tyr
305                 310                 315                 320

Asn Ser Thr Phe Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Thr Lys Gly Gln Pro Arg
        355                 360                 365

Glu Pro Ala Val Tyr Thr Leu Pro Pro Ser Arg Glu Glu Met Thr Lys
    370                 375                 380

Asn Gln Val Lys Leu Val Cys Leu Val Thr Gly Phe Tyr Pro Ser Asp
385                 390                 395                 400

Ile Ala Val Glu Trp Glu Ser Ser Gly Gln Pro Glu Asn Asn Tyr Tyr
                405                 410                 415

Thr Thr Pro Pro Met Leu Asp Ser Asp Gly Ser Phe Ser Leu Val Ser
            420                 425                 430

Trp Leu Asn Val Asp Lys Ser Arg Trp Gln Gln Gly Asn Ile Phe Ser
        435                 440                 445

Cys Ser Val Met His Glu Ala Leu His Asn Arg Phe Thr Gln Lys Ser
    450                 455                 460

Leu Ser Leu Ser Pro Gly
465                 470

<210>  9
<211>  501
<212>  PRT
<213>  Artificial Sequence

<220>
<223>  GBR1342 scFv-Fc sequence: hSP34_H3K21_W100eY/T29E-W91F-L95T
       (hSP34v.3) scFv_11_BTB_D401Q_LALA

<400>  9

Met Arg Ser Pro Ala Gln Leu Leu Phe Leu Leu Leu Leu Trp Ile Pro
1               5                   10                  15

Gly Thr Asn Ala Glu Val Gln Leu Val Glu Ser Gly Gly Gly Leu Val
            20                  25                  30

Gln Pro Gly Gly Ser Leu Arg Leu Ser Cys Ala Ala Ser Gly Phe Thr
        35                  40                  45

Phe Asn Thr Tyr Ala Met Asn Trp Val Arg Gln Ala Pro Gly Lys Gly
    50                  55                  60

Leu Glu Trp Val Ala Arg Ile Arg Ser Lys Tyr Asn Asn Tyr Ala Thr
65                  70                  75                  80

Tyr Tyr Ala Asp Ser Val Lys Gly Arg Phe Thr Ile Ser Arg Asp Asp
                85                  90                  95

Ser Lys Asn Thr Leu Tyr Leu Gln Met Asn Ser Leu Arg Ala Glu Asp
            100                 105                 110

Thr Ala Val Tyr Tyr Cys Val Arg His Gly Asn Phe Gly Asn Ser Tyr
        115                 120                 125

Val Ser Tyr Phe Ala Tyr Trp Gly Gln Gly Thr Thr Val Thr Val Ser
    130                 135                 140

Ser Gly Gly Gly Gly Ser Gly Gly Gly Gly Ser Gly Gly Gly Gly Ser
145                 150                 155                 160

Glu Ile Val Val Thr Gln Ser Pro Ala Thr Leu Ser Val Ser Pro Gly
                165                 170                 175

Glu Arg Ala Thr Leu Ser Cys Arg Ser Ser Thr Gly Ala Val Thr Glu
            180                 185                 190

Ser Asn Tyr Ala Asn Trp Val Gln Glu Lys Pro Gly Gln Ala Phe Arg
        195                 200                 205

Gly Leu Ile Gly Gly Ala Asn Lys Arg Ala Pro Gly Val Pro Ala Arg
    210                 215                 220

Phe Ser Gly Ser Leu Ser Gly Asp Glu Ala Thr Leu Thr Ile Ser Ser
225                 230                 235                 240

Leu Gln Ser Glu Asp Phe Ala Val Tyr Tyr Cys Ala Leu Phe Tyr Ser
                245                 250                 255

Asn Thr Trp Val Phe Gly Gln Gly Thr Lys Leu Glu Ile Lys Gly Gly
            260                 265                 270

Gly Gly Thr Asp Lys Thr His Thr Cys Pro Pro Cys Pro Ala Pro Glu
        275                 280                 285

Ala Ala Gly Gly Pro Ser Val Phe Leu Phe Pro Pro Lys Pro Lys Asp
    290                 295                 300

Thr Leu Met Ile Ser Arg Thr Pro Glu Val Thr Cys Val Val Val Asp
305                 310                 315                 320

Val Ser His Glu Asp Pro Glu Val Lys Phe Asn Trp Tyr Val Asp Gly
                325                 330                 335

Val Glu Val His Asn Ala Lys Thr Lys Pro Arg Glu Glu Gln Tyr Asn
            340                 345                 350

Ser Thr Tyr Arg Val Val Ser Val Leu Thr Val Leu His Gln Asp Trp
        355                 360                 365

Leu Asn Gly Lys Glu Tyr Lys Cys Lys Val Ser Asn Lys Ala Leu Pro
    370                 375                 380

Ala Pro Ile Glu Lys Thr Ile Ser Lys Ala Lys Gly Gln Pro Arg Glu
385                 390                 395                 400

Pro Glu Val Ala Thr Phe Pro Pro Ser Arg Asp Glu Leu Thr Lys Asn
                405                 410                 415

Gln Val Thr Leu Val Cys Leu Val Thr Gly Phe Tyr Pro Ser Asp Ile
            420                 425                 430

Ala Val Glu Trp Glu Ser Asn Gly Gln Pro Glu Asn Asn Tyr Lys Thr

435                 440                 445

Asp Pro Pro Leu Leu Glu Ser Gln Gly Ser Phe Ala Leu Ser Ser Arg
    450                 455                 460

Leu Arg Val Asp Lys Ser Arg Trp Gln Gln Gly Asn Val Phe Ser Cys
465                 470                 475                 480

Ser Val Met His Glu Ala Leu His Asn His Tyr Thr Gln Lys Ser Leu
                485                 490                 495

Ser Leu Ser Pro Gly
            500

<210>  10
<211>  723
<212>  PRT
<213>  Artificial Sequence

<220>
<223>  GBR1372_SF3_scFV_Hc sequence

<400>  10

Met Glu Thr Asp Thr Leu Leu Leu Trp Val Leu Leu Leu Trp Val Pro
1               5                   10                  15

Gly Ser Thr Gly Glu Val Gln Leu Val Glu Ser Gly Gly Gly Leu Val
            20                  25                  30

Gln Pro Gly Gly Ser Leu Arg Leu Ser Cys Ala Ala Ser Gly Phe Thr
        35                  40                  45

Phe Asn Thr Tyr Ala Met Asn Trp Val Arg Gln Ala Pro Gly Lys Gly
    50                  55                  60

Leu Glu Trp Val Ala Arg Ile Arg Ser Lys Tyr Asn Asn Tyr Ala Thr
65                  70                  75                  80

Tyr Tyr Ala Asp Ser Val Lys Ser Arg Phe Thr Ile Ser Arg Asp Asp
                85                  90                  95

Ser Lys Asn Thr Leu Tyr Leu Gln Met Asn Ser Leu Arg Ala Glu Asp
            100                 105                 110

Thr Ala Val Tyr Tyr Cys Val Arg His Gly Asn Phe Gly Asn Ser Tyr
        115                 120                 125

Val Ser Tyr Phe Ala Tyr Trp Gly Gln Gly Thr Thr Val Thr Val Ser
    130                 135                 140

Ser Gly Gly Gly Gly Ser Gly Gly Gly Gly Ser Gly Gly Gly Gly Ser
145                 150                 155                 160

Glu Ile Val Val Thr Gln Ser Pro Ala Thr Leu Ser Val Ser Pro Gly
                165                 170                 175

Glu Arg Ala Thr Leu Ser Cys Arg Ser Ser Thr Gly Ala Val Thr Glu
            180                 185                 190

Ser Asn Tyr Ala Asn Trp Val Gln Glu Lys Pro Gly Gln Ala Phe Arg
        195                 200                 205

Gly Leu Ile Gly Gly Ala Asn Lys Arg Ala Pro Gly Val Pro Ala Arg
    210                 215                 220

Phe Ser Gly Ser Leu Ser Gly Asp Glu Ala Thr Leu Thr Ile Ser Ser
225                 230                 235                 240

Leu Gln Ser Glu Asp Phe Ala Val Tyr Tyr Cys Ala Leu Phe Tyr Ser
                245                 250                 255

Asn Thr Trp Val Phe Gly Gln Gly Thr Lys Leu Glu Ile Lys Gly Gly
            260                 265                 270

Gly Gly Thr Gln Val Gln Leu Gln Glu Ser Gly Pro Gly Leu Val Lys
        275                 280                 285

Pro Ser Glu Thr Leu Ser Leu Thr Cys Thr Val Ser Gly Gly Ser Val
    290                 295                 300

Ser Ser Gly Asp Tyr Tyr Trp Thr Trp Ile Arg Gln Ser Pro Gly Lys
305                 310                 315                 320

Gly Leu Glu Trp Ile Gly His Ile Tyr Tyr Ser Gly Asn Thr Asn Tyr
                325                 330                 335

Asn Pro Ser Leu Lys Ser Arg Leu Thr Ile Ser Ile Asp Thr Ser Lys
            340                 345                 350

Thr Gln Phe Ser Leu Lys Leu Ser Ser Val Thr Ala Ala Asp Thr Ala
        355                 360                 365

Ile Tyr Tyr Cys Val Arg Asp Arg Val Thr Gly Ala Phe Asp Ile Trp
    370                 375                 380

Gly Gln Gly Thr Met Val Thr Val Ser Ser Ala Ser Thr Lys Gly Pro

385                 390                 395                 400

Ser Val Phe Pro Leu Ala Pro Ser Ser Lys Ser Thr Ser Gly Gly Thr
                405                 410                 415

Ala Ala Leu Gly Cys Leu Val Lys Asp Tyr Phe Pro Glu Pro Val Thr
            420                 425                 430

Val Ser Trp Asn Ser Gly Ala Leu Thr Ser Gly Val His Thr Phe Pro
        435                 440                 445

Ala Val Leu Gln Ser Ser Gly Leu Tyr Ser Leu Ser Ser Val Val Thr
    450                 455                 460

Val Pro Ser Ser Ser Leu Gly Thr Gln Thr Tyr Ile Cys Asn Val Asn
465                 470                 475                 480

His Lys Pro Ser Asn Thr Lys Val Asp Lys Lys Val Glu Pro Lys Ser
                485                 490                 495

Cys Asp Lys Thr His Thr Cys Pro Pro Cys Pro Ala Pro Glu Ala Ala
            500                 505                 510

Gly Gly Pro Ser Val Phe Leu Phe Pro Pro Lys Pro Lys Asp Thr Leu
        515                 520                 525

Met Ile Ser Arg Thr Pro Glu Val Thr Cys Val Val Val Asp Val Ser
    530                 535                 540

His Glu Asp Pro Glu Val Gln Phe Lys Trp Tyr Val Asp Gly Val Glu
545                 550                 555                 560

Val His Asn Ala Lys Thr Lys Pro Arg Glu Glu Gln Tyr Asn Ser Thr
                565                 570                 575

Phe Arg Val Val Ser Val Leu Thr Val Leu His Gln Asp Trp Leu Asn
            580                 585                 590

Gly Lys Glu Tyr Lys Cys Lys Val Ser Asn Lys Ala Leu Pro Ala Pro
        595                 600                 605

Ile Glu Lys Thr Ile Ser Lys Thr Lys Gly Gln Pro Arg Glu Pro Ala
    610                 615                 620

Val Tyr Thr Leu Pro Pro Ser Arg Glu Glu Met Thr Lys Asn Gln Val
625                 630                 635                 640

Lys Leu Val Cys Leu Val Thr Gly Phe Tyr Pro Ser Asp Ile Ala Val
                645                 650                 655

Glu Trp Glu Ser Ser Gly Gln Pro Glu Asn Asn Tyr Tyr Thr Thr Pro
            660                 665                 670

Pro Met Leu Asp Ser Asp Gly Ser Phe Ser Leu Val Ser Trp Leu Asn
        675                 680                 685

Val Asp Lys Ser Arg Trp Gln Gln Gly Asn Ile Phe Ser Cys Ser Val
    690                 695                 700

Met His Glu Ala Leu His Asn Arg Phe Thr Gln Lys Ser Leu Ser Leu
705                 710                 715                 720

Ser Pro Gly

<210>  11
<211>  234
<212>  PRT
<213>  Artificial Sequence

<220>
<223>  GBR1372_SF3_Lc

<400>  11

Met Arg Ser Pro Ala Gln Leu Leu Phe Leu Leu Leu Leu Trp Ile Pro
1               5                   10                  15

Gly Thr Asn Ala Asp Ile Gln Met Thr Gln Ser Pro Ser Ser Leu Ser
            20                  25                  30

Ala Ser Val Gly Asp Arg Val Thr Ile Thr Cys Gln Ala Ser Gln Asp
        35                  40                  45

Ile Ser Asn Tyr Leu Asn Trp Tyr Gln Gln Lys Pro Gly Lys Ala Pro
    50                  55                  60

Lys Leu Leu Ile Tyr Asp Ala Ser Asn Leu Glu Thr Gly Val Pro Ser
65                  70                  75                  80

Arg Phe Ser Gly Ser Gly Ser Gly Thr Asp Phe Thr Phe Thr Ile Ser
                85                  90                  95

Ser Leu Gln Pro Glu Asp Ile Ala Thr Tyr Phe Cys Gln His Phe Asp
            100                 105                 110

His Leu Pro Leu Ala Phe Gly Gly Gly Thr Lys Val Glu Ile Lys Arg

115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  12
<211>  246
<212>  PRT
<213>  Artificial Sequence

<220>
<223>  GBR1372_SF3_Fc

<400>  12

Met Glu Thr Asp Thr Leu Leu Leu Trp Val Leu Leu Leu Trp Val Pro
1               5                   10                  15

Gly Ser Thr Gly Asp Lys Thr His Thr Cys Pro Pro Cys Pro Ala Pro
            20                  25                  30

Glu Ala Ala Gly Gly Pro Ser Val Phe Leu Phe Pro Pro Lys Pro Lys
        35                  40                  45

Asp Thr Leu Met Ile Ser Arg Thr Pro Glu Val Thr Cys Val Val Val
    50                  55                  60

Asp Val Ser His Glu Asp Pro Glu Val Lys Phe Asn Trp Tyr Val Asp
65                  70                  75                  80

Gly Val Glu Val His Asn Ala Lys Thr Lys Pro Arg Glu Glu Gln Tyr
                85                  90                  95

Asn Ser Thr Tyr Arg Val Val Ser Val Leu Thr Val Leu His Gln Asp
            100                 105                 110

Trp Leu Asn Gly Lys Glu Tyr Lys Cys Lys Val Ser Asn Lys Ala Leu
        115                 120                 125

Pro Ala Pro Ile Glu Lys Thr Ile Ser Lys Ala Lys Gly Gln Pro Arg
    130                 135                 140

Glu Pro Glu Val Ala Thr Phe Pro Pro Ser Arg Asp Glu Leu Thr Lys
145                 150                 155                 160

Asn Gln Val Thr Leu Val Cys Leu Val Thr Gly Phe Tyr Pro Ser Asp
                165                 170                 175

Ile Ala Val Glu Trp Glu Ser Asn Gly Gln Pro Glu Asn Asn Tyr Lys
            180                 185                 190

Thr Asp Pro Pro Leu Leu Glu Ser Asp Gly Ser Phe Ala Leu Ser Ser
        195                 200                 205

Arg Leu Arg Val Asp Lys Ser Arg Trp Gln Gln Gly Asn Val Phe Ser
    210                 215                 220

Cys Ser Val Met His Glu Ala Leu His Asn His Tyr Thr Gln Lys Ser
225                 230                 235                 240

Leu Ser Leu Ser Pro Gly
                245